# EUROPÄISCHE PATENTSCHRIFT

(11) **EP 1 196 196 B1**
(45) Veröffentlichungstag und Bekanntmachung des Hinweises auf die Patenterteilung: **08.09.2010**
(21) Anmeldenummer: 00954335.6
(22) Anmeldetag: 14.07.2000
(51) Int. Cl.: A61K 47/48

(54) **KONJUGAT ZUR VERMITTLUNG EINES ZELL-, KOMPARTIMENT- ODER MEMBRANSPEZIFISCHEN TRANSPORTS VON WIRKSUBSTANZEN**
CONJUGATE FOR MEDIATING CELL, COMPARTMENT OR MEMBRANE-SPECIFIC TRANSPORT OF ACTIVE SUBSTANCES
CONJUGUE DESTINE A LA MISE EN OEUVRE D'UN TRANSPORT DE SUBSTANCES ACTIVES SPECIFIQUE SELON LES CELLULES, COMPARTIMENTS OU MEMBRANES

(30) Priorität: 16.07.1999 DE 19933492
(43) Veröffentlichungstag der Anmeldung: 17.04.2002
(73) Patentinhaber: Deutsches Krebsforschungszentrum Stiftung des öffentlichen Rechts, 69120 Heidelberg (DE)
(72) Erfinder: BRAUN, Klaus, 69207 Sandhausen (DE); PESCHKE, Peter, 67459 Böhl-Iggelheim (DE); FRIEDRICH, Eckart, 76831 Landau-Ilbesheim (DE); PIPKORN, Rüdiger, 69120 Heidelberg (DE); WALDECK, Waldemar, 69514 Laudenbach (DE); DEBUS, Jürgen, 76698 Stettfeld (DE)
(74) Vertreter: Schüssler, Andrea
(86) Internationale Anmeldenummer: PCT/DE2000/002346
(87) Internationale Veröffentlichungsnummer: WO 2001/005432

(56) Entgegenhaltungen:
- WO-A-00/01417
- WO-A-00/58488
- WO-A-97/12912
- WO-A-99/57138
- WO-A-99/57138
- FR-A- 2 786 397
- FR-A- 2 787 793
- PIETERSZ, GEOFFREY A. (1) ET AL: "A 16-mer peptide ( RQIKIWFQNRRMKWKK ) from antennapedia preferentially targets the Class I pathway." VACCINE, (8 JANUARY, 2001) VOL. 19, NO. 11-12, PP. 1397-1405. PRINT. , XP001002158
- MI Z ET AL: "Characterization of a class of cationic peptides able to facilitate efficient protein transduction in vitro and in vivo." MOLECULAR THERAPY, (2000 OCT) 2 (4) 339-47. , XP001004549
- DEROSSI D (REPRINT) ET AL: "Trojan peptides: the penetratin system for intracellular delivery" TRENDS IN CELL BIOLOGY,ELSEVIER SCIENCE LTD,XX, Bd. 8, Februar 1998 (1998-02), Seiten 84-87, XP002122131 ISSN: 0962-8924
- ROUSELLE C. ET AL: "New advances in the transport of doxorubicin through the blood-brain barrier by a peptide vector-mediated strategy" MOLECULAR PHARMACOLOGY, Bd. 57, April 2000 (2000-04), Seiten 679-686, XP001004535
- POOGA M. ET AL: "Cell penetrating PNA constructs regulate galanin receptor levels and modify pain transmission in vivo" NATURE BIOTECHNOLOGY, Bd. 16, September 1998 (1998-09), Seiten 857-861, XP001002100
- BRAUN ET AL.: "A biological transporter for the delivery of peptide nucleic acids (PNAs) to the nuclear compartment of living cells." J. MOL. BIOL., Bd. 318, 2002, Seiten 237-243, XP002248667
- BRAUN ET AL.: "A biological transporter for the delivery of peptide nucleic acids (PNAs) to the nuclear compartment of living cells." J. MOL. BIOL., Bd. 318, 2002, Seiten 237-243, XP002248667

## Beschreibung

Die vorliegende Erfindung betrifft Konjugate zur Vermittlung eines zell-, kompartiment- oder membranspezifischen Transports von Wirksubstanzen. Weiter betrifft die Erfindung Verfahren zur Herstellung dieser Konjugate sowie deren Verwendung.

Es ist bekannt, daß zelluläre Membransysteme weitestgehend impermeabel für viele Stoffe (z.B. Nukleinsäuren, Proteine, chemische Substanzen) sind, die von außen in die zelle eingebracht werden sollen. Zum Einbringen von Nukleinsäuren können Zellmembranen durch physikalische Prozesse (Transfektion bei Eukaryonten, Transformation bei Prokaryonten) und biologische Vorgänge (Infektion) überwunden werden. Der Transformation, d.h. dem unmittelbaren Aufnehmen der nackten Nukleinsäure durch die Zelle, geht eine Behandlung der Zellen voraus. Unterschiedliche Methoden zur Erzeugung dieser "kompetenten Zellen" stehen zur Verfügung. Die meisten Verfahren basieren auf den Beobachtungen von Mandel und Higa (J. Mol. Biol. 53, S. 159-162 (1970)), die erstmals zeigen konnten, daß die Ausbeuten bei der Aufnahme von Lambda-DNA durch Bakterien in Gegenwart von Calciumchlorid ganz wesentlich gesteigert werden. Diese Methode ist erstmals von Cohen et al. (proc. Natl. Acad. Sci. USA 69, S. 2210-2114 (1972)) für Plasmid-DNA erfolgreich eingesetzt und durch viele Modifikationen verbessert worden. Eine andere Transformationsmethode beruht auf der Beobachtung, daß hochfrequente Wechselstromfelder Zellmembranen aufbrechen können (Elektroporation). Diese Technik läßt sich ausnutzen, um nackte DNA nicht nur in prokaryontische Zellen, sondern auch in eukaryontische Zellsysteme einzuschleusen (Weaver et al., J. Cell Biochem. 51, S. 426-435 (1993)). Zwei sehr sanfte Methoden zur DNA-Einbringung in eukaryontische Zellen wurden von Sikes et al. (Hum. Gen. Therap. 5, S. 837-840 (1994)) bzw. Yang et al. (Proc. Natl. Acad. Sci USA 87, S. 9568-9572 (1990) entwickelt. Sie beruhen auf der direkten Injektion der DNA in einzelne Zellen (Mikroinjektion) bzw. auf dem Beschuß einer Zellpopulation mit Mikroprojektilen aus Wolfram, an deren Oberfläche die betreffende Nukleinsäure gebunden wurde ("Gene gun"). Parallel zur physikalischen Transformation von Zellen haben sich biologische Infektionsmethcdcn bewährt. Dazu zählen insbesondere die virale Einbringung von Nukleinsäuren in Zellen (Chatterjee et al., Science 258, S.1485-1486 (1992); Cossett and Rusell, Gene Therapy 3, S. 946-956 (1996); Bilbao et al., FASEB J. 11, S. 624-634 (1997)) sowie die über Liposomen vermittelte Lipofektion (Bennett et al., J. Drug Targeting 5, S. 149-162 (1997)). Weiter sind Standardmethoden des liposomalen Transports (Gao and Huang, Gene Therapy 2, S. 710-722 (1995); Akhtar et al., Nucl. Acid. Res. 19, S. 5551-5559 (1991)) und die Poly-L-Lysinierung (Leonetti et al., Bioconj. Chem. 1(2), S. 149 (1990) von Wirksubstanzen zu nennen, um diese in Zellen einschleusen zu können.

Derossi et al. (Trends in Cell Biology, Bd. 8 (1998-02), S. 84-87) beschreiben Penetratin als Trägermoleküle, die die Plasmamembran überwinden können, um exogene Makromoleküle, z.B. Oligonukleotide oder Oligopeptide, in lebende Zellen einzuschleusen. Ferner werden in der US 5 677 274 Transportfusionspeptide bestehend aus einer Translokationsdomäne für die Plasmamembran und einer Ligandendomäne, die spezifisch an einen Rezeptor an der Zelloberfläche bindet, beschrieben. Die Ligandendomäne bindet an die zu erkennende Zielstruktur an der Zelloberfläche und die Translokationsdomäne bewirkt anschließend den Transport eines Wirkstoffs in die von der Ligandendomäne erkannten Zelle. US 5,877,282 offenbart Polypeptide aus einem Transportvermittler, einem Adresspeptid für den Zellkern (NLS) und einer zweiten NLS-Sequenz und WO 97/28822 offenbart Konjugate aus Insulin einem Adresspeptid und Chlorin e6, wobei der Transport in die Zelle durch Rezeptorvermittelte Endozytose erfolgt. Ferner offenbart US 5,674,977 Konjugate, die ein Transportpeptid (CTS), ein Adresspeptid (NLS) sowie einen Wirkstoff (Acr) aufweisen.

Trotz der oben aufgezählten Vielzahl von Methoden, die zellulären Membransysteme zu überwinden, gibt es noch keine universelle Methode, um unterschiedliche Wirkstoffe in Zellen hineinzubringen. Alle aufgeführten physikalischen und biochemischen Methoden sind artifiziell und unphysiologisch, insofern als sie nicht zellimmanente Mechanismen nutzen. Viren als Transportmittel sind zum gegenwärtigen Stand immer noch nicht sicher frei von Toxizität und oft auch nicht effektiv. Sie werden auch vom Immunsystem erkannt.

Die Aufgabe der vorliegenden Erfindung bestand deshalb darin, eine Möglichkeit zu entwickeln, die das gerichtete und spezifische Einschleusen von Wirkstoffen in Zellen und Kompartimente erlaubt. Es sind dabei folgende Anforderungen zu verfüllen:
- universelle Anwendbarkeit
- zell-, kompartiment- und membranspezifisches Einschleusungsverhalten
- hohe Effektivität
- geringe Immunogenität
- Minimierung des Infektionsrisikos
- ausreichend lange Verweildauer

Gelöst wird diese Aufgabe durch die Gegenstände der Patentansprüche.

Von den Erfindern wurde ein Konjugat entwickelt, das die folgenden Komponenten aufweist:
- einen Transportvermittler für die Zellmembran ("P"),
- ein kompartiment - oder membranspezifisches Adressprotein bzw. -peptid ("AP"),
- einen zu transportierenden Wirkstoff ("W"); und
- eine Redoxkopplung zwischen P und AP

Der Aufbau des erfindungsgemäßen Konjugats ist:

P-AP-W

ganz bevorzugt mit einem Spacer ("SP") :

P - AP - BP - W

Den Transportvermittler für die zellmembran (vorstehend mit "P" abgekürzt) stellt ein Peptid bzw. Protein dar, das die Plasmamembran überwinden kann. Die Länge dieses Peptids bzw. Proteins unterliegt keiner Beschränkung, solange es die obige Eigenschaft aufweiset. Beispiele für "P" stammen vorzugweise aus der Penetratin-Familie (Derossi et al., 1998, Trends Cell Biol. 8 , S. 84-87) oder sind Transportan bzw. Teile davon (Pooga et al., The Faseb Journal (1998), Vol. 12, S. 68 ff.), wobei solche aus der Penetratin-Familie bevorzugt sind. Ein Beispiel für "P" stellt ein Penetratin mit der folgenden Sequenz dar:
NH₂-RQIKIWFQNRRMKWKK-(NH₂-Arg-Gln-Ile-Lys-Ile-Trp-Phe-Gln-Asn-Arg-Arg-Met-Lys-Trp-Lys-Lys-)

Weitere Beispiele für das Transportprotein "P" sind:

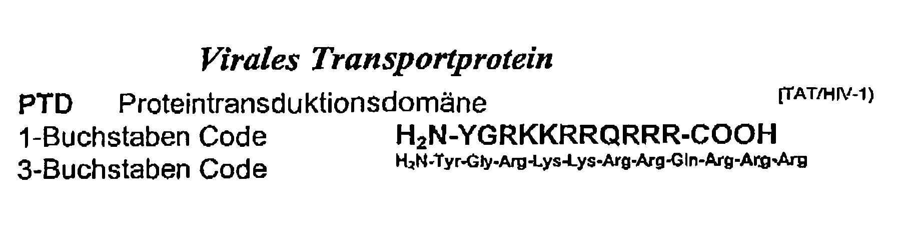

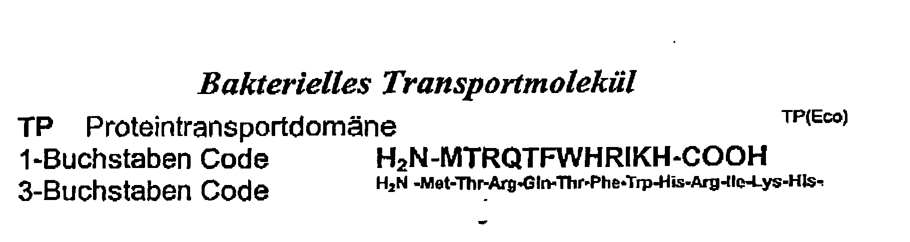

Hergestellt wird die ausgewählte "P"-Sequenz auf biologischem Weg (Reinigung natürlicher Transportvermittlerproteine oder Klonierung und Expression der Sequenz in einem eukaryotischen oder prokaryotischen Expressionssystem), bevorzugt aber auf synthetischem Weg, z.B. nach der etablierten Merrifield-Methode (Merrifield, J. Am. Chem. Soc. 85: 2149, 1963).

Die Auswahl des Adressproteins bzw. -peptids (vorstehend mit "AP" abgekürzt) hängt davon ab, welche Membran bzw. welches Membransystem überwunden und welches Zielkompartiment der Zelle (Cytoplasma, Zellkern, Mitochondrien, Chloroplast, Endoplasmatisches Retikulum) oder der Zellorganelle erreicht werden soll. Die Länge dieses Adresspeptids bzw. -proteins unterliegt keiner Beschränkung, solange es die Eigenschaft aufweist, einen kompartiment- oder membranspezifischen Transport zu gewährleisten. Für die Einbringung von Wirkstoffen, insbesondere Nukleinsäuren, werden im allgemeinen "AP" ausgewählt, die ein kompartiment- oder membranspezifisches Erkennungssignal enthalten und dadurch den angehängten Wirkstoff an seinen Wirkort dirigieren. Zur Auswahl stehen "AP", die Wirkstoffe in Gegenwart oder Abwesenheit eines Membranpotentials transportieren können. Grundsätzlich ist für den Transport in das Zellkompartiment die reine Adressequenz ausreichend. Es können aber auch "AP" ausgewählt werden, die über eine zell- oder kompartimentspezifische Peptidasespaltstelle verfügen. Diese Spaltstelle liegt im günstigsten Fall innerhalb der Signalsequenz, kann aber auch an diese durch zusätzliche Aminosäuren angefügt werden, um nach Erreichen des Zielkompartiments das Abspalten der Adressequenz sicherzustellen. Hergestellt wird die ausgewählte "AP"-Sequenz auf biologischem (Reinigung natürlicher Transportvermittlerproteine oder Klonierung und Expression der Sequenz in einem eukaryontischen oder prokaryontischen Expressionssystem), bevorzugt aber auf synthetischem Weg, z.B. nach der etablierten Merrifield-Methode (Merrifield, J. Am. Chem. Soc. 85: 2149, 1963). Beispiele für Adressproteine bzw. peptide sind:
- Import in das ER: H₃N⁺-Met-Met-Ser-Phe-Val- Ser-Leu-Leu-Leu-Val-Gly- Ile-Leu-Phe-Trp-Ala-Thr- Glu-Ala-Glu-Gln-Leu-Thr- Lys-Cys-Glu-Val-Phe-Gln-
   - Reimport in das ER: H₂N-Lys-Asp-Glu-Leu-COO
   - Import in Mitochondrien: H₃N⁺-Met-Leu-Ser-Leu-Arg- Gln-Ser-Ile-Arg-Phe-Phe- Lys-Pro-Ala-Thr-Arg-Thr- Leu-Cys-Ser-Ser-Arg-Tyr- Leu-Leu
   - Import in den Zellkern: -Pro-Pro-Lys-Lys-Lys-Arg- Lys-Val H₃N⁺-Pro-Lys-Lys-Lys-Arg- Lys-Val- (= Nuclear localisation sequence aus SV40-T-Antigen;)
   - Import in Peroxisomen: H₂N-Ser-Lys-Leu-COO-

   - Bindung an die Zellmembran: H₃N⁻-Gly-Ser-Ser-Lys-Ser- Lys-Pro-Lys-

Weiter kann das Konjugat ggf. einen Spacer (vorstehend mit "SP" abgekürzt) enthalten, der sich vorzugsweise zwischen dem Adressprotein/-peptid und dem zu transportierenden Wirkstoff befindet. Er kann aber zusätzlich oder alternativ auch zwischen dem Transportvermittler und dem Adressprotein vorliegen. Der Spacer dient dazu, ggf. vorhandene sterische Wechselwirkungen zwischen den komponenten aufzuheben bzw. günstig zu beeinflussen. Der Spacer kann beispielsweise ausgewählt sein aus: Polylysin. Polyethylenglykol (PEG), Derivate der Poly-Methacrylsäure oder Polyvinylpyrrolidon (PVP) .

Zwischen dem Transportvermittler und dem Adressprotein/-peptid ist eine Redoxspaltstelle, z.B. -Cystein-S-S-Cystein-O-N-H-. Die zwischen Transportvermittler und Adressprotein entstehende Bindung ist eine Redoxkopplung (schonende zellimmanente Verknüpfung mittels DMSO; Rietsch und Beckwith, 1998, Annu. Rev. Gent 32, s. 163-84):

Cystein-SH SH-Cystein → Cystein-S-S-Cystein

Der wirkstoff bzw. die Wirksubstanz (vorstehend mit "W" abgekürzt) unterliegt keinerlei Beschränkungen. Er ist frei wählbar, je nach Wunsch, welche Wirkung in einer Zelle erzeugt werden soll. Der Wirkstoff kann ein Diagnostikum und/oder Therapeutikum sein. Es kann auch mehr als ein Wirkstoff in dem Konjugat vorhanden sein. Der Wirkstoff kann ggf. markiert sein, z.B, radioaktiv, mit einem Farbstoff, mit Biotin/Avidin usw. Der Wirkstoff kann eine Nukleinsäure, ein Protein bzw. Peptid, eine chemische Substanz usw. sein. Beispielsweise seien genannt: cDNA, genomische DNA, vollständige Gene, regulatorische Elemente, Transkriptionsfaktoren, Molekularsonden, Oligonukleotide, mRNA, mTRNA, Antisense-RNA, Antisense-Oligonukleotide, Plasmide, virale DNA, synthetische Nukleotide, PNA (Peptide Nucleic Acids), einzelne Aminosäuren und deren Derivate, Peptide, Proteine, monoklonale und/oder polyklonale Antikörper, pharmazeutische Wirkstoffe, Chemotherapeutika, Farbstoffe, Sensibilisatoren, Partikel.

Die Synthese der Konjugatbestandteile "P" und "AP" erfolgt vorzugsweise synthetisch nach der Merrifield-Methode (Merrifield, J. Am. Chem. Soc. 85: 2149, 1963) Die Ankopplung der anderen Bestandteile (z.B. Spacer und/oder Wirkstoff) daran erfolgt durch kovalente chemische Bindung. Die Einfügung der Redoxspaltstelle zwischen "P" und "AP" erfolgt auf chemischen Wege durch die oben erwähnte Redoxkopplung. Auch zwischen einem ggf. vorhandenen Spacer und dem Wirkstoff bzw. dem Adressprotein und dem Wirkstoff liegt eine kovalente Bindung vor, bevorzugt eine Säureamid-Bindung. Mögliche Alternativen sind Ether- oder Ester-Bindungen, je nach den in der zu konjugierenden Substanz vorhandenen funktionellen Gruppe(n).

Das Konjugat wird bevorzugt in folgenden Schritten aufgebaut:
1) Getrennte Peptidsynthese von "P", "AP" und ggf. des Spacers (z.B. nach der Merrifield-Methode)
2) Kovalente Verknüpfung zwischen "AP" und Wirkstoff, ggf. mit einem Spacer dazwischen,
3) Redoxkopplung des Produkts aus Schritt 2) mit "P" mittels Redoxkopplung (z.B. in Wasser/DMSO)
4) Reinigung (z.B. mittels HPLC)

Die erfindungsgemäßen Konjugate haben den Vorteil, unabhängig von Art und Größe eines Wirkstoffs, diesen in Zellen einbringen und in das gewünschte Zellkompartiment transportieren zu können. Es ist somit eine Verbesserung in Diagnostik und Therapie in Human- und Tiermedizin sowie eine Anwendung in der wissenschaftlichen Forschung zu erwarten. Insbesondere die Gentherapie kann durch die erfindungsgemäßen Konjugate einen Aufschwung erwarten, da vollständige Gene einschließlich ihrer regulatorischen Elemente transportierbar werden. Aber auch alle anderen Wirkstoffe lassen sich mit den erfindungsgemäßen Konjugaten spezifischer an den Wirkort bringen, was das Auftreten von unerwünschten Nebenwirkungen reduziert. Es wurde gefunden, daß Konjugate bis 25 MDa in das Zellinnere einzubringen sind. Darüber hinaus kommt es oftmals zur Auslösung von Apoptose, was durchaus ein gewünschter Effekt sein kann. Die erfindungsgemäßen Konjugate zeichnen sich durch eine universelle Einsetzbarkeit aufgrund ihres zell-, kompartiment- und membranspezifischen Einschleusungsverhaltens aus.

Die Erfindung wird weiter anhand der beigefügten Figuren beschrieben:
- Fig. 1:: erfindungsgemäßes Konjugat
- Fig. 2:: Generelles Schema der Fmoc-Synthese
- Fig. 3:: Ergebnisse der Fluoreszenzkorrelationsspektroskopie- Messung an AT1-Zellen
A) Konjugat-Konzentration: 50 nM Inkubationszeit: 5 Std.
B) Konjugat-Konzentration: 5 nM Inkubationszeit: 5 Std.
C) Konjugat-Konzentration: 50 nM Inkubationszeit: 24 Std.
D) Konjugat-Konzentration: 5 nM Inkubationszeit: 24 Std.
- Fig. 4:: Konzentrations- und zeitabhängiger Transport von ^{Rhodamin110} (L)-Penetratin/RPMI-Medium. DU145-Zellen: Inkubation mit 20 µM und 100 pM Endkonzentration

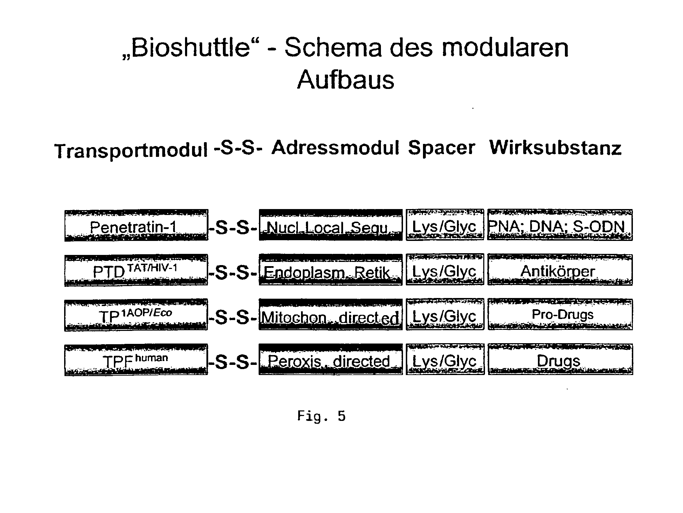

- Fig. 5:: Beispiele für erfindungsgemäße Konjugate
- Fig. 6:: Herstellung von PNA-Konstrukten
- Fig. 7:: Hemmung der Proliferation von AT-1 Zellen durch Einbringeneines anti-sense Konstrukts

Die Erfindung wird weiter anhand der nachfolgenden Beispiele beschrieben.

### Beispiel 1: Konjugat aufweisend einen Penetratin-Bestandteil, eine NLS, einen Polylysin-Spacer und Rhodamin

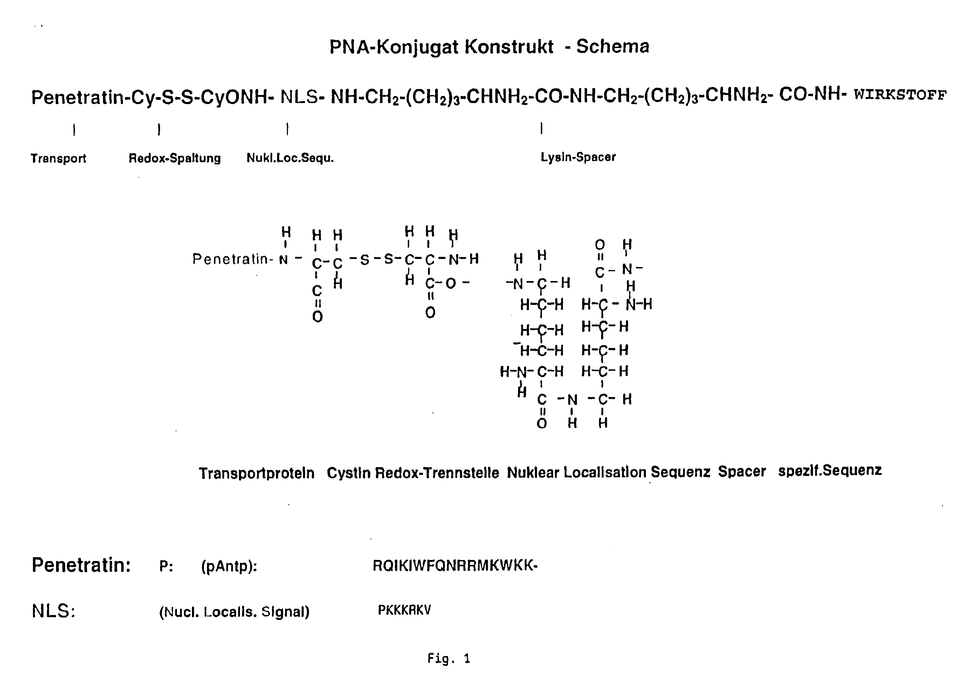

Zum Aufbau des Konjugats wird auf Fig. 1 verwiesen.

| | |
|---|---|
| Penetratin: | NH₂-RQIKIWFQNRRMKWKK- |
| NLS (Nuclear Localisation Sequence): | NH₂-PKKKRKV |
| Spacer (= (Lys)₂): | NH-CH₂-(CH₂)₃-CHNH₂-CO-NH-CH₂-(CH₂)₃- CHNH₂-CO-NH |

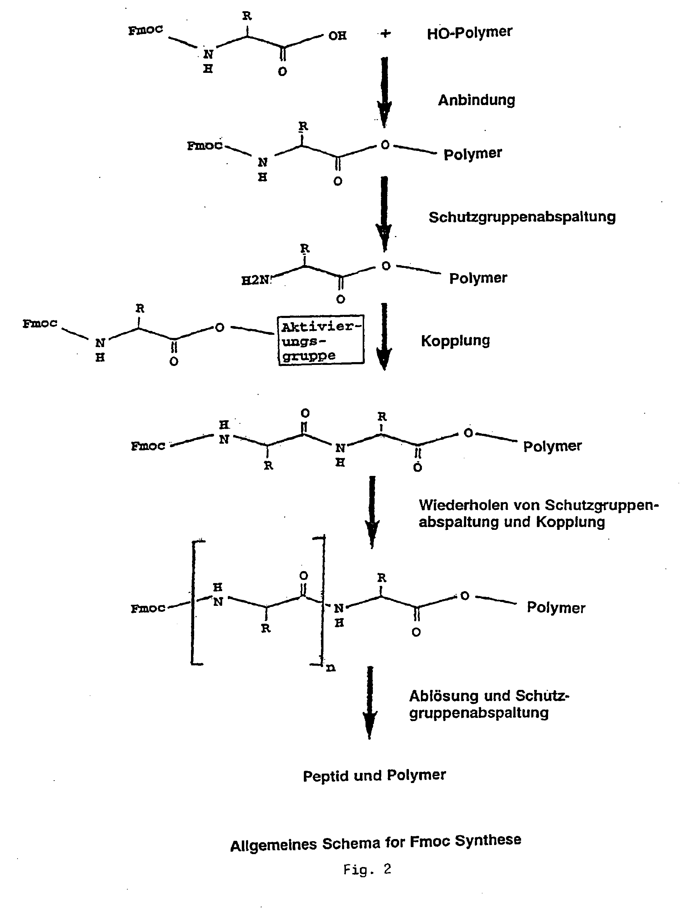

Die Penetratinsequenz, die NLS und der Spacer wurden nach der Standard Fmoc-Methode ("Peptide", H.-D. Jakubke, Chemie und Biologie Spektrum, Akad. Verl. 1996, ISBN 3-8274-0000-7) getrennt synthetisiert. Das generelle Schema der Fmoc-Synthese ist in Fig. 2 gezeigt. Zur Synthese der verschiedenen Komponenten-Sequenzen wird zuerst die erste Fmoc Aminosäure (käuflich erhältlich von Fa. Calbiochem GmbH, D-65796 Bad Soden) an ein unlösliches Polystyrol-Trägerharz über einen säurelabilen Linker (= para-Benzyl-oxybenzyl-alkohol-handle) angefügt. Die Abspaltung der Schutzgruppe wird durch Behandlung des Harzes mit 20% Piperidin in Dimethylformamid erreicht. Die zweite Fmoc-Aminosäure wird unter Verwendung einer präaktivierten Spezies (z.B. in den Aminosäure-Einzelbestandteilen vorhandenen Succinimid-, Pentafluorphenylester- oder p-Nitrophenylestergruppen) oder in-situ Aktivierung angekoppelt, jeweils nachdem von der vorhergehenden Aminosäure die Schutzgruppe durch basische Behandlung entfernt worden ist. Jede weitere Aminosäure wird analog angekoppelt. Nachdem das gewünschte Peptid synthetisiert worden ist, wird dieses mittels Behandlung mit 95% Trifluoressigsäure (TFA) + 5% Scavenger (z.B. Triethylsilan) von dem Träger entfernt und die Schutzgruppen abgespalten. Die entstandenen Roh-Peptide werden durch präparative HPLC auf einer YMC ODS-A 7A S-5µm Umkehrphasensäule (20 x 250 mm) unter Verwendung eines Elutionsmittels enthaltend 0,1 % Trifluoressigsäure in Wasser (A) bzw. 60% wässrigem Acetonitril (B) gereinigt. Die Peptide wurden mit einem sukzessiven linearen Gradienten von 25% B bis 60% B in 40 Minuten bei einer Fließgeschwindigkeit von 10 ml/min. eluiert. Die den gereinigten Peptiden entsprechenden Fraktionen wurden lyophilisiert.

Die aufgereinigten Peptid-Komponenten werden gemeinsam mit 20%iger wässriger DMSO-Lösung über 5 Stunden bei Raumtemperatur behandelt, wodurch eine oxidative Kopplung der Komponenten resultiert. An den Spacer wird als zu transportierende Wirksubstanz, z.B. Rhodamin 110, gekoppelt. Dies erfolgt durch Säureamid-Kopplung an der freien α-Aminogruppe des Lysinspacers. Das komplette Konjugat wird anschließend mittels Umkehrphasen-HPLC gereinigt.

Analog wurden die weiteren erfindungsgemäßen Konjugate hergestellt:

^{Alexa™}(*L*)-PTD^{(TAT/HIV-1)}-S-S-(*L*)-NLS-KK^{(Rhodamin110)}-PNA

^{Alexa™}(*L*)-TP^{(1A0P/*ECo*)}-S-S-(*L*)-NLS-KK^{(Rhodamin110)}-PNA

PNA = NH₂-TTA AGG AGG CTC-COOH (Beispiel für Wirkstoff) Alexa 350 = Farbstoff (Fa. Molecular Probes, USA)

### Beispiel 2: Einbringen eines erfindungsgemäßen Konjugats in Zellen

AT-1 (Ratten-Prostata-Carcinoma) und DU-145 (menschliche Prostata-Carcinoma, ATCC HTB-81) Zellen wurden in RPMI 1640 kultiviert, ergänzt mit 10% FCS, 2 mM Glutamin, 100 U/min. Penicillin, 100 µg/ml Streptomycin.

Zur Fluoreszenzkorrelationsspektroskopie (FCS) läßt man AT-1 bzw. DU-145 Zellen auf Objekträgern 24 Std. anwachsen. Nach Mediumwechsel mit farbstoffreiem RPMI 1640 (ohne Phenolrot) wird das Penetratin-enthaltende Konjugat von Beispiel 1 (100 nM) mit RPMI auf die Zellen gegeben und 5, 24 bzw. 48 Std. bei 37°C und 5% CO₂ inkubiert. Anschließend wird das Konjugat-enthaltende Medium entfernt und zweimal mit 200 µl farbstoffreiem RPMI gewaschen und anschließend per FCS gemessen. Die Anregung mit dem Laser erfolgt bei 488 nm und die Emission bei 538 nm.

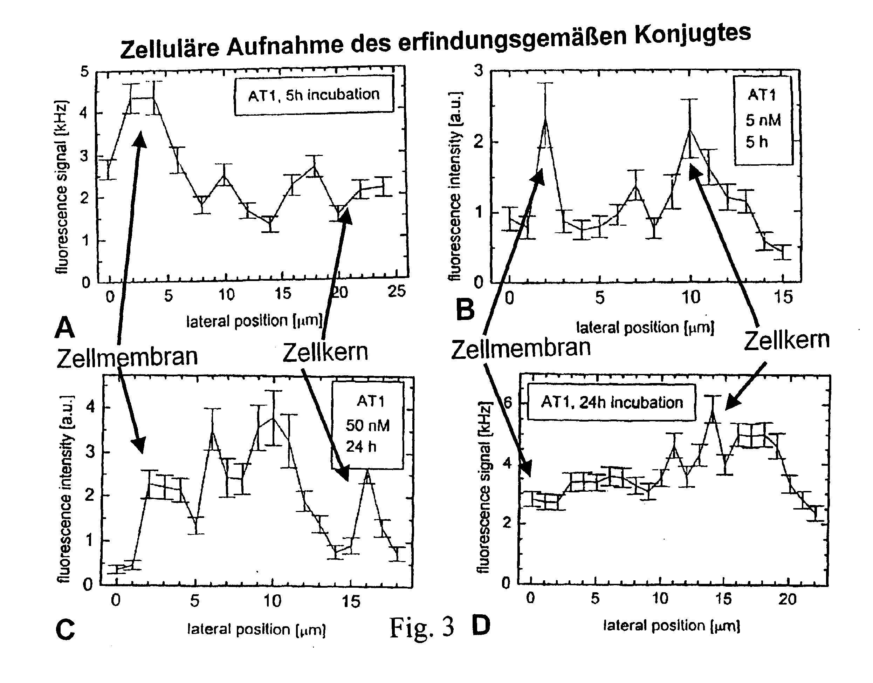

Das Konjugat wird auf dem Weg in den Zellkern verfolgt. Dazu wird unter dem Lichtmikroskop eine Zelle ausgewählt focusiert. Nach Focusierung und Justierung des Lasers wird in 100 µm Schritten durch die Zellen gefahren, und die Fluoreszenz wird in Form von Lichtblitzen über Photomultiplier gemessen. Dabei wandern große Moleküle und kleine Moleküle unterschiedlich schnell. Erfaßt wird die Anzahl der diffundierenden Moleküle in einem Bereich von jeweils 100 µm. So läßt sich mit der Signaldauer die Größe der diffundierten Moleküle bestimmen. Die dazugehörige Grafik ist in Fig. 3 gezeigt.

In einem weiteren Experiment wird die Kinetik, mit der das Konjugat ins Cytoplasma gelangt, mit dem gleichen Verfahren ermittelt. Die AT-1 Zellen wurden wieder 24 Std. adhäriert. Das das Konjugat enthaltende Medium wurde wie vorhergehend beschrieben eingesetzt. Allerdings wurde jetzt sofort das Fluoreszenzsignal mit der FCS gemessen.

Die FCS zeigte deutlich eine starke Anreicherung an der Zellmembran nach 5 Stunden Inkubationszeit. Eine Diffusion war nicht zu erkennen. Nach 24 Std. Inkubationszeit zeigten sich nur noch geringfügige Mengen an Konjugat in der Zellmembran. Auffällig war jetzt eine Anreicherung im Kern, die sich im Beobachtungszeitraum von 48 Std. noch verstärkte.

Zur Kontrolle wurden Konjugate eingesetzt, bei denen Rhodamin 110 entweder nur an Pentratin oder an NLS gebunden war. Diese zeigten nicht den vorstehend beschriebenen Effekt der Zellkernanreicherung. Die Konjugate wurden, falls sie überhaupt den Übertritt in die Zelle schafften, an der Zellmembran bzw. der Nuclearhülle angehalten und reicherten sich dort an.

Analog wie vorstehend beschrieben wurden alle in Beispiel 1 hergestellten Konjugate hinsichtlich ihres zeitabhängigen intrazellulären Transports in das Zytoplasma (Z) bzw. den Zellkern (N) untersucht. Die Inkubationszeiten waren jedoch abweichend von den vorstehend angegebenen Inkubationszeiten 1, 3, 6, 10 und 24 Stunden. Die Ergebnisse sind in der Tabelle 1 gezeigt.

### Beispiel 3: Konzentrationsabhängiger Transport

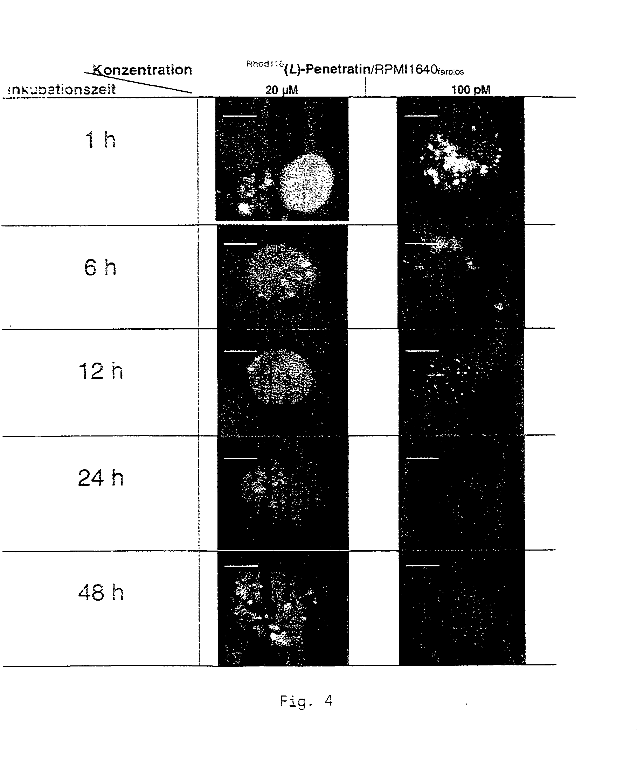

Es sollte untersucht werden, inwieweit die Konzentration des Transportpeptids ^{Rhodamin110}(L)-Penetratin/RPMI-Medium den zellulären und kerngerichteten Transport auch zeitabhängig beeinflussen. Verglichen wurde die Fluoreszenz von 20 µM und 100 pM Endkonzentration ^{Rhodamin110}(L)-Penetratin/RPMI-Medium. Dazu wurden DU-145 Zellen 1, 6, 12, 24 und 48 Stunden mit den angebenen Konzentrationen inkubiert. Anschließend wird dreimal mit RPMI (ohne Penetratin), einmal mit PBS und nochmals mit RPMI. Nach Versehen der Zellen mit Objektträgerdeckeln wurde umgehend die Fluoreszenz mittels der CLSM (Konfokalen Laser Scanning Mikroskopie) bestimmt. Die Ergebnisse sind in Fig. 4 gezeigt. Daraus ist ersichtlich, daß mit einer hohen Konzentration von über 20 µM ein unspezifischer Transport stattfindet, was auf Zytotoxizität hindeutet. In einer darunterliegenden Konzentration dagegen findet ein spezifischer Transport ins Zytoplasma statt.

### Beispiel 4: Hemmung der Proliferation von AT-1 Zellen durch Einbringen eines Anti-sense Konstrukts

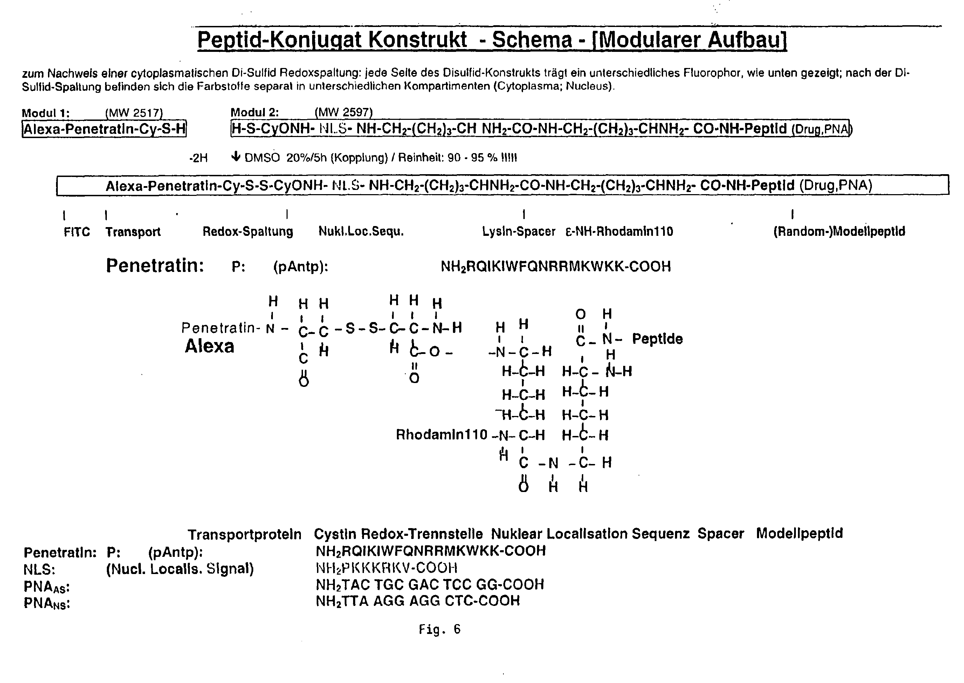

Es wurden unter analoger Anwendung der in Beispiel 1 beschriebenen Methode Peptid-Konjugat-Konstrukte gemäß Fig. 6 hergestellt. Dabei war der Wirkstoff einmal ein PNA mit der Sequenz NH₂-TAC TGC GAC TCC GG-COOH (anti-sense zu Ratten P2-Promotor c-myc = PNA_{AS}) und einmal eine Non-sense ("random) Sequenz mit der Nukleotidabfolge NH₂-TTA AGG AGG CTC-COOH (=PNA_{NS})_{.}

AT-1 Zellen wurden in RPMI 1640 kultiviert, ergänzt mit 10% FCS, 2 mM Glutamin, 100 U/min. Penicillin, 100 µg/ml Streptomycin.

Man läßt AT-1 Zellen auf Objekträgern 24 Std. anwachsen. Nach Mediumwechsel mit farbstoffreiem RPMI 1640 (ohne Phenolrot) werden die Konjugate (100 nM) jeweils mit RPMI auf die Zellen gegeben und 24, 48, 72 bzw. 96 Std. bei 37°C und 5% CO₂ inkubiert. Anschließend wird das Konjugat-enthaltende Medium entfernt und zweimal mit 200 µl farbstoffreiem RPMI gewaschen. Es erfolgt mittels der Coulter-Counting-Methode die Bestimmung der Zellzahl von AT-1 Zellen.

Als Kontrolle wurden unbehandelte AT-1 Zellen verwendet. Eine weitere Kontrolle stellt unligiertes PNA_{AS} dar. Diese Kontrollen wurde analog wie vorstehend beschrieben mit den AT-1 Zellen inkubiert.

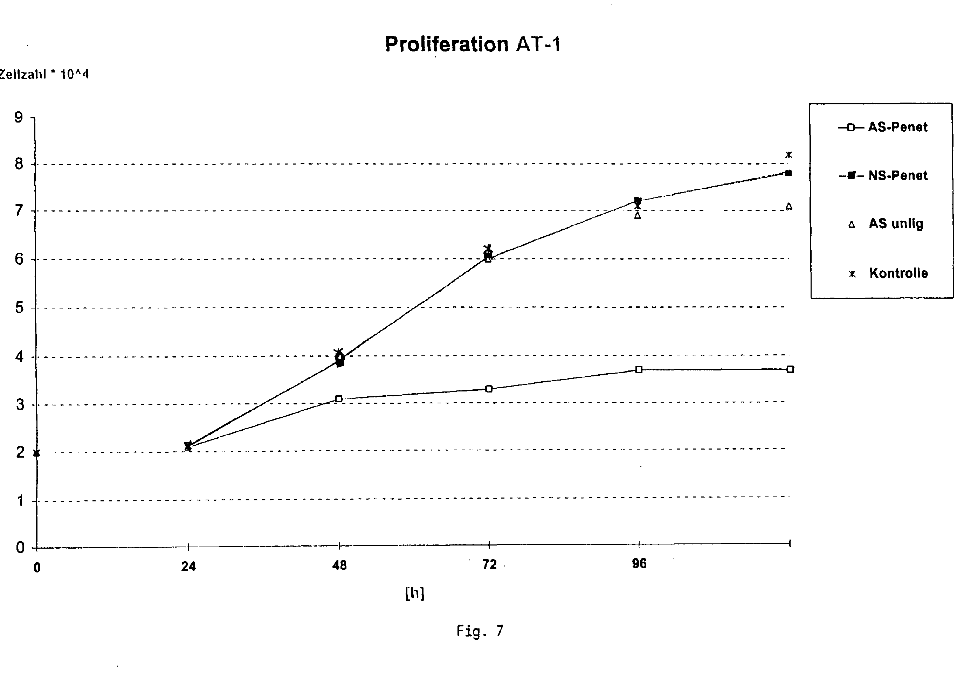

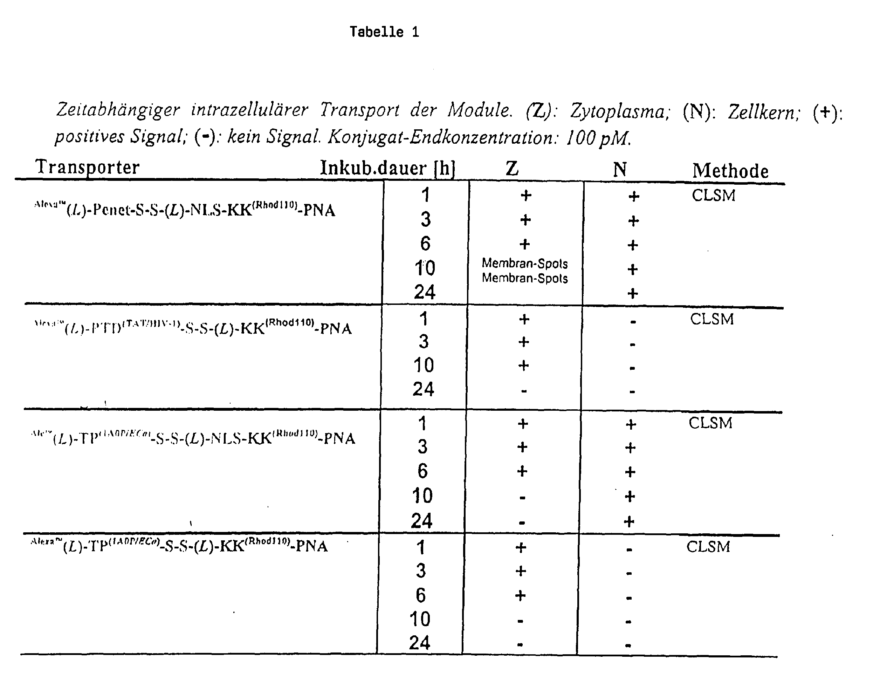

Das Ergebnis dieses Experiments ist in Fig. 7 gezeigt. Eine Hemmung der Proliferation von AT-1 erfolgte nur nach Verabreichung des Anti-sense Konstrukts, d.h. hiermit ist klar gezeigt, daß nur mittels des erfindungsgemäßen Konstrukts ein Eintritt in den Zellkern erfolgt und die Anti-sense Sequenz die gewünschte Wirkung dort entfalten kann. Unligierte Antisense Sequenz ist genauso wirkungslos wie die Kontrolle oder ein Konstrukt, das nicht mit einer der AT-1 Sequenzen hybridisieren kann.

## Patentansprüche

1. Konjugat zur Vermittlung eines zell-, kompartiment- oder membranspezifischen Transports, wobei das Konjugat die folgenden Komponenten aufweist:
- ein Transportpeptid oder protein für die Zellmembran, das die Plasmamembran überwinden kann;
- ein kompartiment- oder membranspeziiisches Adressprotein bzw. - peptid, das eine Addresssequenz für ein Zielkompartiment der Zelle oder eine Zellorganelle aufweist, und
- einen zu transportierenden Wirkstoff,
**dadurch gekennzeichnet, dass**
das Konjugat die Struktur Transportpeptid bzw. -protein - Adressprotein - Wirkstoff hat und das Transportpeptid oder -protein mit dem Adressprotein bzw. -peptid durch eine Redoxkopplung verbunden ist, wobei die Redoxkopplung Cystein-S-S-Cystein ist.

2. Konjugat nach Anspruch 1, wobei das Transportpeptid bzw. -protein aus der Penetratin-Familie stammt, Transportan oder die Proteintransduktionsdomäne des TAT-Proteins von HIV-1 ist,

3. Konjugat nach Anspruch 1, wobei das Transportpeptid ein bakterielles Transportpeptid ist.

4. Konjugat nach Anspruch 2, wobei eines der Penetratine folgende Sequenz hat:
NH₂-RQIKIWFQNRRMKWKK-

5. Konjugat nach einem der vorhergehenden Ansprüche, wobei das zell-, kompartiment- oder membranspezifische Adressprotein bzw. -peptid ausgewählt ist aus:
für Import in das ER H₃N⁺-Met-Met-Ser-Phe-Val- Ser-Leu-Leu-Leu-Val-Gly- Ile-Leu-Phe-Trp-Ala-Thr- Glu-Ala-Glu-Gln-Leu-Thr- Lys-Cys-Glu-Val-Phe-Gln-
für Reimport in das ER H₂N-Lys-Asp-Glu-Leu-COO
für Import in Mitochondrien H₃N⁺-Met-Leu-Ser-Leu-ArgGln-Ser-Ile- Arg-Phe-Phe-Lys-Pro-Ala-Thr-Arg-Thr- Leu-Cys-Ser-Ser-Arg-Tyr-Leu-Leu
für Import in den Zellkern -Pro-Pro-Lys-Lys-Lys-Arg-Lys-Val H₃N⁺-Pro-Lys-Lys-Lys-Arg-Lys-Val- (= Nuclear localisation sequence aus SV40- T-Antigen)
für Import in Peroxisomen H₂N-Ser-Lys-Leu-COO
für Bindung an die Zellmembran H₃N⁺-Gly-Ser-Ser-Lys-Ser-Lys-Pro-Lys-

6. Konjugat nach Anspruch 5, wobei die Sequenz für den Import in den Zellkern folgende Sequenz hat:
H₃N⁺-Pro-Lys-Lys-Lys-Arg-Lys-Val-

7. Konjugat nach einem der vorhergehenden Ansprüche, wobei der Wirkstoff ein Diagnostikum oder Therapeutikum ist.

8. Konjugat nach Anspruch 7, wobei der Wirkstoff ausgewählt ist aus Nukleinsäuren, Proteinen/Peptiden und/oder chemischen Substanzen.

9. Konjugat nach einem der vorhergehenden Ansprüche, wobei ein Spacer vorhanden ist, um sterische Wechselwirkungen zwischen den Komponenten aufzuheben oder günstig zu beeinflussen.

10. Konjugat nach Anspruch 9, wobei sich der Spacer zwischen dem Adressprotein und dem Wirkstoff befindet.

11. Konjugat nach Anspruch 9, wobei der Spacer Polylysin, Polyethylenglykol oder Polyvinylpyrrolidon ist.

12. Konjugat nach einem der vorhergehenden Ansprüche, wobei das Konjugat modular aufgebaut ist.

13. Verfahren zur Herstellung eines Konjugats nach einem der Ansprüche 1-12 aufweisend die folgenden Schritte:
1) Getrennte Peptidsynthese von Transportprotein bzw. -peptid, Adressprotein bzw. -peptid und ggf. des Spacers
2) Kovalente Verknüpfung zwischen Adressprotein bzw. -peptid und Wirkstoff, ggf. mit einem Spacer dazwischen,
3) Redoxkopplung des Produkts aus Schritt 2) mit Transportprotein bzw. -peptid mittels Redoxkopplung, wobei die Redoxkopplung Cystein-S-S-Cystein ist.

14. Verfahren nach Anspruch 13, wobei die Peptidsynthese gemäß der Merrified-Methode durchgeführt wird.

15. Verfahren nach Anspruch 13 oder 14, wobei die Redoxkopplung in einer wässrigen DMSO-Lösung durchgeführt wird.

16. Verfahren nach einem der Ansprüche 13-15, wobei sich noch ein Reinigungsschritt anschließt.

17. Verfahren nach Anspruch 16, wobei die Reinigung mittels HPLC stattfindet.

18. Konjugat nach einem der Ansprüche 1-12 zur Verwendung als Arzneimittel.

19. Verwendung eines Konjugats nach einem der Ansprüche 1-12 zum Herstellen eines Mittels für die Diagnose und/oder Therapie.

## Claims

1. A conjugate for mediating a cell, compartment or membrane specific transport, the conjugate comprising the following components:
- a transport peptide or protein for the cell membrane which can overcome the plasma membrane;
- a compartment or membrane specific address protein or peptide which has an address sequence for a target compartment of the cell or a cell organelle, and
- an active substance to be transported,
**characterized in that**
the conjugate has the structure of transport peptide or protein - address protein
- active substance and the transport peptide or protein is linked with the address protein or peptide by a redox coupling, the redox coupling being cysteine-S-S-cysteine.

2. The conjugate according to claim 1, wherein the transport peptide or protein is derived from the penetratin family, is transportan or the protein transduction domain of the TAT protein of HIV-1.

3. The conjugate according to claim 1, wherein the transport peptide is a bacterial transport peptide.

4. The conjugate according to claim 2, wherein one of the penetratins has the following sequence:
NH₂-RQIKIWFQNRRMKWKK-.

5. The conjugate according to any of the preceding claims, wherein the cell, compartment or membrane specific address protein or peptide is selected from:
for import into the ER H₃N⁺-Met-Met-Ser-Phe-Val- Ser-Leu-Leu-Leu-Val-Gly- Ile-Leu-Phe-Trp-Ala-Thr- Glu-Ala-Glu-Gln-Leu-Thr- Lys-Cys-Glu-Val-Phe-Gln-
for reimport into the ER H₂N-Lys-Asp-Glu-Leu-COO⁻
for import into mitochondria H₃N⁺-Met-Leu-Ser-Leu-Arg-Gln-Ser-Ile Arg-Phe-Phe-Lys-Pro-Ala-Thr-Arg-Thr- Leu-Cys-Ser-Ser-Arg-Tyr-Leu-Leu
for import into the nucleus -Pro-Pro-Lys-Lys-Lys-Arg-Lys-Val H₃N⁺-Pro-Lys-Lys-Lys-Arg-Lys-Val- (= nuclear localization sequence from SV40-T antigen)
for import into peroxisomes H₂N-Ser-Lys-Leu-COO⁻
for binding to the cell membrane H₃N⁺-Gly-Ser-Ser-Lys-Ser-Lys-Pro-Lys-

6. The conjugate according to claim 5, wherein the sequence for the import into the nucleus has the following sequence:
H₃N⁺-Pro-Lys-Lys-Lys-Arg-Lys-Val-.

7. The conjugate according to any of the preceding claims, wherein the active substance is a diagnostic agent or therapeutic agent.

8. The conjugate according to claim 7, wherein the active substance is selected from nucleic acids, proteins/peptides and/or chemical substances.

9. The conjugate according to any of the preceding claims, wherein a spacer is present to eliminate or favorably influence steric interactions between the components.

10. The conjugate according to claim 9, wherein the spacer is located between the address protein and the active substance.

11. The conjugate according to claim 9, wherein the spacer is polylysine, polyethylene glycol or polyvinyl pyrrolidone.

12. The conjugate according to any of the preceding claims, wherein the conjugate has a modular structure.

13. A method of preparing a conjugate according to any of claims 1 to 12, comprising the steps of:
1) separate peptide synthesis of transport protein or peptide, address protein or peptide and optionally spacer,
2) covalent linkage between address protein or peptide and active substance, optionally with a spacer in between,
3) redox coupling of the product of step 2) with transport protein or peptide by means of redox coupling, the redox coupling being cysteine-S-S-cysteine.

14. The method according to claim 13, wherein the peptide synthesis is carried out according to the Merrifield method.

15. The method according to claim 13 or 14, wherein the redox coupling is carried out in an aqueous DMSO solution.

16. The method according to any of claims 13 to 15, which is followed by a purification step.

17. The method according to claim 16, wherein the purification is carried out by means of HPLC.

18. A conjugate according to any of claims 1 to 12 for use as a medicament.

19. Use of a conjugate according to any of claims 1 to 12 for the preparation of a product for diagnosis and/or therapy.

## Revendications

1. Conjugué destiné à la mise en oeuvre d'un transport spécifique selon les cellules, compartiments ou membranes, le conjugué présentant les composants suivants:
- un peptide ou une protéine de transport pour la membrane de cellule pouvant vaincre la membrane de plasma;
- une protéine ou un peptide d'adressage spécifique selon les compartiments ou membranes qui présente une séquence d'adressage pour un compartiment cible de la cellule ou une organelle de cellule, et
- une substance active à transporter,
**caractérisé par le fait que**
le conjugué a la structure peptide ou protéine de transport - protéine d'adressage
- substance active et que le peptide ou la protéine de transport est lié à la protéine ou au peptide d'adressage par un couplage rédox, le couplage rédox étant cystéine-S-S-cystéine.

2. Conjugué selon la revendication 1, dans lequel le peptide ou la protéine de transport est issu de la famille de pénétratine, est transportane ou les domaines de transduction de protéine de la protéine TAT de HIV-1.

3. Conjugué selon la revendication 1, dans lequel le peptide de transport est un peptide de transport bactérien.

4. Conjugué selon la revendication 2, dans lequel la pénétratine a la séquence suivante:
NH₂-RQIKIWFQNRRMKWKK-

5. Conjugué selon l'une des revendications précédentes, dans lequel la protéine ou le peptide d'adressage spécifique selon les cellules, compartiments ou membranes est choisi parmi:
pour importation dans l'ER H₃N⁺-Met-Met-Ser-Phe-Val- Ser-Leu-Leu-Leu-Val-Gly- Ile-Leu-Phe-Trp-Ala-Thr- Glu-Ala-Glu-Gln-Leu-Thr- Lys-Cys-Glu-Val-Phe-Gln-
pour réimportation dans l'ER H₂N-Lys-Asp-Glu-Leu-COO
pour importation dans les mitochondries H₃N⁺-Met-Leu-Ser-Leu-ArgGln-Ser-Ile- Arg-Phe-Phe-Lys-Pro-Ala-Thr-Arg-Thr- Leu-Cys-Ser-Ser-Arg-Tyr-Leu-Leu
pour importation dans le noyau de cellule -Pro-Pro-Lys-Lys-Lys-Arg-Lys-Val H₃N⁺-Pro-Lys-Lys-Lys-Arg-Lys-V al- (=séquence de localisation nucléaire de l'antigène T SV40)
pour importation dans les peroxisomes H₂N-Ser-Lys-Leu-COO
pour liaison à la membrane de cellule H₃N⁺-Gly-Ser-Ser-Lys-Ser-Lys-Pro-Lys-

6. Conjugué selon la revendication 5, dans lequel la séquence pour l'importation dans le noyau de cellule a la séquence suivante:
H₃N⁺-Pro-Lys-Lys-Lys-Arg-Lys-Val-

7. Conjugué selon l'une des revendications précédentes, dans lequel la substance active est une substance diagnostique ou une substance thérapeutique.

8. Conjugué selon la revendication 7, dans lequel la substance active est choisie parmi les acides nucléiques, les protéines/peptides et/ou des substances chimiques.

9. Conjugué selon l'une des revendications précédentes, dans lequel est présent un espaceur, pour éliminer ou influencer favorablement les interactions stériques entre les composants.

10. Conjugué selon la revendication 9, dans lequel l'espaceur se trouve entre la protéine d'adressage et la substance active.

11. Conjugué selon la revendication 9, dans lequel l'espaceur est polylysine, polyéthylène-glycol ou polyvinylpyrrolidone.

12. Conjugué selon l'une des revendications précédentes, dans lequel le conjugué est construit de manière modulaire.

13. Procédé pour préparer un conjugué selon l'une des revendications 1 à 12, présentant les étapes suivantes:
1) synthèse de peptide séparée de la protéine ou du peptide de transport, de la protéine ou du peptide d'adressage et éventuellement de l'espaceur,
2) couplage covalent entre la protéine ou le peptide d'adressage et la substance active, éventuellement avec un espaceur entre eux,
3) couplage rédox du produit de l'étape 2) avec la protéine ou le peptide de transport au moyen d'un couplage rédox, le couplage rédox étant cystéine-S-S-cystéine.

14. Procédé selon la revendication 13, dans lequel la synthèse de peptide est réalisée selon la méthode de Merrifield.

15. Procédé selon la revendication 13 ou 14, dans lequel le couplage rédox est réalisé dans une solution de DMSO aqueuse.

16. Procédé selon l'une des revendications 13 à 15, dans lequel suit encore une étape de purification.

17. Procédé selon la revendication 16, dans lequel la purification a lieu au moyen de HPLC.

18. Conjugué selon l'une des revendications 1 à 12, destiné à être utilisé comme médicament.

19. Utilisation d'un conjugué selon l'une des revendications 1 à 12 pour la préparation d'un moyen de diagnostic et/ou de thérapie.
